# EUROPÄISCHE PATENTANMELDUNG

(11) **EP 2 113 514 A1**
(43) Veröffentlichungstag der Anmeldung: **04.11.2009**
(21) Anmeldenummer: 08008396.7
(22) Anmeldetag: 03.05.2008
(51) Int. Cl.: C07K 14/47, C12N 15/82, C12P 21/00, A61K 38/17

(54) **Verfahren zur Erzeugung von hypoallergenen Glykoproteinen in mutierten oder transgenen Pflanzen oder Pflanzenzellen sowie mutierte oder transgene Pflanzen und Pflanzenzellen zur Erzeugung hypoallergener Glykoproteine**

(71) Anmelder: Westfälische Wilhelms-Universität Münster, 48149 Münster (DE)
(72) Erfinder: von Schaewen, Antje Prof.Dr., 48161 Münster (DE); Kaulfürst-Soboll, Heidi, 59387 Ascheberg (DE)
(74) Vertreter: Stoppkotte, Cornelia

(57) **Zusammenfassung**

Die vorliegende Erfindung betrifft ein Verfahren zur Erzeugung von hypoallergenen Glykoproteinen in mutierten oder transgenen Pflanzen, Teilen dieser Pflanzen oder daraus hergestellten Pflanzenzellen sowie die entsprechenden mutierten oder transgenen Pflanzen, Pflanzenteile und Pflanzenzellen, bei welchen die Aktivität des Enzyms Golgi α-Mannosidase II beseitigt oder verringert ist und welche hypoallergene Glykoproteine produzieren.

## Beschreibung

Die vorliegende Erfindung betrifft ein Verfahren zur Erzeugung von hypoallergenen Glykoproteinen in mutierten oder transgenen Pflanzen, Teilen dieser Pflanzen oder daraus hergestellten Pflanzenzellen sowie die entsprechenden mutierten oder transgenen Pflanzen, Pflanzenteile und Pflanzenzellen.

In allen Eurkaryoten ist *N*-Glykosylierung im Sekretorischen System ein essentieller Prozess. Glykoproteine werden zunächst im Endoplasmatischen Retikulum (ER) zusammengesetzt, wobei membrangebundene Glykane (Dolicholpyrophosphat-Oligosaccharide) cotranslational auf spezielle Asparagin-Reste in der wachsenden Polypeptidkette transferiert werden. In höheren Organismen unterliegen Zuckereinheiten, die an der Oberfläche der gefalteten Polypeptidkette liegen, in Golgi-Zisternen weiteren Trimm- und Modifikationsreaktionen. Durch verschiedene Glykosidasen und Glykosyltransferasen im ER und Golgi-Apparat (die Ausstattung kann Spezies-abhängig unterschiedlich sein), werden zunächst typische Glc₃Man₉GlcNAc₂-Grundeinheiten ("core"-Glykane) des "high"-Mannose-Typs (Anglizismen werden in der vorliegenden Anmeldung in Anführungsstriche gesetzt) gebildet und anschließend bei Passage durch die verschiedenen Golgi-Zisternen in sogenannte "komplexe" Glykane umgewandelt. Letztere zeichnen sich durch eine geringere Zahl an Mannose-Einheiten und den Besitz weiterer Zuckerreste, wie Fucose und/oder Xylose sowie Galaktose in Pflanzen bzw. Sialinsäure (*N*-Acetylneuraminsäure, NeuNAc) in Säugern, aus.

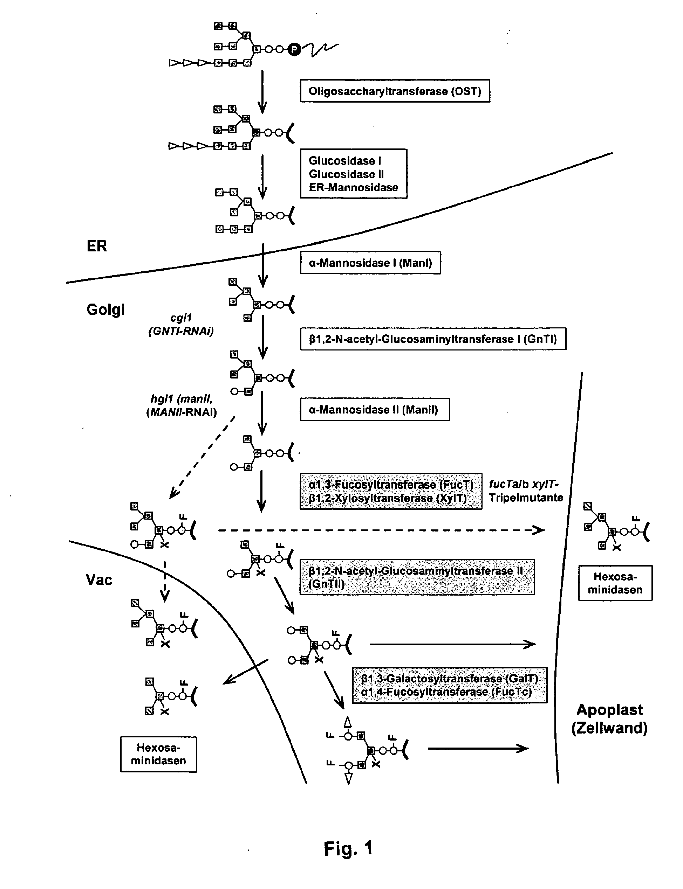

In Höheren Pflanzen umfasst die Bildung komplexer *N*-Glykane im Sekretorischen System insgesamt acht Schritte (Fig. 1), wobei die Verknüpfung mit "core" α1,3-Fucose- und β1,2-Xylose-Resten für pflanzliche Glykoproteine charakteristisch ist. Da diese Modifikationen zwar auch in einigen Invertebraten vorkommen, in Säugern aber nur α1,6-verknüpfte "core"-Fucose-Reste existieren, wirken pflanzliche Glykoproteine auf Letztere immunogen. Dies zeigt eindrucksvoll, dass nicht nur die Zuckerreste als solche, sondern auch die Art ihrer Verknüpfung über Bindung oder Nicht-Bindung von Antikörpern entscheidet.

Glykoproteine sind für Medizin und Forschung von großer Bedeutung. Eine Isolierung von Glykoproteinen im Großmaßstab ist jedoch aufwendig und teuer. Die direkte Anwendung konventionell isolierter Glykoproteine ist oft problematisch, da einzelne Reste der Glykan-Komponenten bei Verabreichung als Therapeutikum ungewünschte Nebenwirkungen auslösen können. In den meisten Fällen kann auf die Glykan-Komponente nicht verzichtet werden, da sie vor allem zu den physikochemischen Eigenschaften (wie Faltung, Stabilität und Löslichkeit) der Glykoproteine beiträgt.

Hefen haben sich zur Gewinnung von Glykoproteinen für Medizin und Forschung größtenteils als ungeeignet erwiesen, da sie nur Glykosylierungen zum sogenannten "high"-Mannose-Typ durchführen können. Insekten und Höhere Pflanzen zeigen zwar "komplexe", aber von Tieren abweichende Glykoprotein-Modifikationen, so dass aus diesen Organismen isolierte Glykoproteine in Säugern immunogen wirken (Faye & Chrispeels, 1988; Strasser et al., 2008). Tierische Organismen mit Glykosylierungsdefekten sind meist nicht lebensfähig, da die terminalen Glykan-Reste (beispielsweise membranständiger Glykoproteine) biologische Signalfunktion besitzen und vor allem für die Zell-Zell-Erkennung während der Embryonalentwicklung unentbehrlich sind. Es existieren zwar Säugerzell-Linien (CHO, "Chinese Hamster Ovary cells") mit definierten Glykosylierungsdefekten, jedoch ist deren Kultivierung arbeitsintensiv und kostspielig.

Im Einzelnen wurden für Säuger auf Zellkulturebene bereits unterschiedliche Glykosylierungsmutanten beschrieben. Diese Mutanten sind entweder in der Biosynthese reifer Oligosaccharidketten am Dolicholpyrophosphat oder in der Glykan-Prozessierung betroffen bzw. zeigen Abweichungen in ihren terminalen Zuckerresten. Einige dieser Zell-Linien weisen einen konditional-letalen Phänotyp auf oder zeigen Defekte im intrazellulären Proteintransport.

Die Folgen dieser Defekte für den intakten Organismus sind schwer abschätzbar. Es wurde beobachtet, dass eine Veränderung im Muster komplexer Glykane auf Zelloberflächen von Säugern mit Tumor- und Metastasenbildung einhergeht (Li et al., 2008 und darin zitierte Arbeiten). Glykosylierungsmutanten kommen in Säugern daher sehr selten vor. Unter der Abkürzung HEMPAS ("Hereditary Erythroblastic Multinuclearity with a Positive Acidified Serum lysis test") zusammengefasste Defekte beruhen entweder auf einem Defizit von α-Mannosidase II im Golgi-Apparat bzw. in Lysosomen und/oder niedrigen Gehalten des Enzyms *N*-Acetyl-Glucosaminyltransferase II (GnTII) und wirken sich stark einschränkend auf die Lebensfähigkeit der mutierten Organismen aus (Fukuda, 1990). In "knock out"-Mäusen, in denen Golgi α-Mannosidase II (GMII) zerstört ist, wird ein alternativer Syntheseweg beschritten, so dass sie lebensfähig aber anämisch sind (Chui et al., 1997; Moremen, 2002). Patienten mit Mutationen in GnTII leiden an CDGSII ("carbohydrate deficient glycoprotein syndrome type II") mit schweren, multiplen Entwicklungsdefekten (Tan et al., 1996). "knock out"-Mäuse, in denen das Gen für *N*-Acetyl-Glucosaminyltransferase I (GnTI) zerstört wurde, sterben bereits im Embryonalstadium an multiplen Entwicklungsdefekten (Ioffe & Stanley, 1994; Metzler et al., 1994).

Aufgrund der Ergebnisse mit Säugern bzw. Säugerzellen (aufwendig und anfällig für Kontamination mit humanen Pathogenen) wurden in den letzten Jahren vermehrt Versuche unternommen, heterologe Glykoproteine in Pflanzen oder Pflanzenzellen zu erzeugen, die so modifiziert sind, dass sie Glykoproteine mit verminderten immunogenen Eigenschaften synthetisieren.

So wurden in früheren Arbeiten Arabidopsis-Mutanten isoliert (von Schaewen et al., 1993), die im zweiten Schritt der Glykan-Modifkation im Golgi-Apparat defekt sind, so dass Glykoproteine mit einheitlicher Man₅GlcNAc₂-Modifikation akkumulieren. Trotz fehlender Aktivität der *N-*Acetyl-Glukosaminyltransferase I (NAG oder GnTI) unterschieden sich diese Arabidopsis *cgl1-*Mutanten unter standardisierten Anzucht-Bedingungen (d.h. in Klimakammern oder im Gewächshaus) nicht merklich vom Wiltyp. *GNTI*-codierende cDNA-Sequenzen aus Arabidopsis, Kartoffel und Tabak wurden erfolgreich zur "antisense"-Drosselung komplexer Glykoprotein-Glykane in Solanaceen eingesetzt (Wenderoth & von Schaewen, 2000). Dies war ein erster Hinweis darauf, dass auch agronomisch wichtige Kulturpflanzen fehlende GnTI-Aktivität prinzipiell tolerieren und zur Produktion modifizierter Glykoproteine eingesetzt werden könnten.

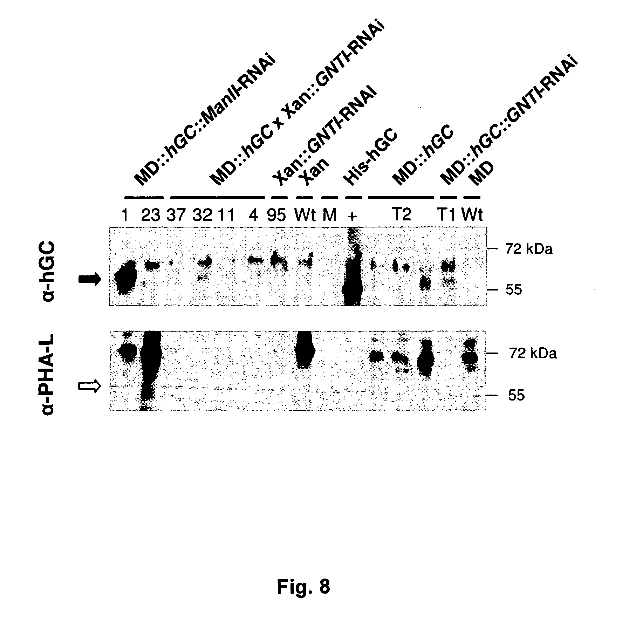

Auf dieser Basis wurde dann versucht, ein Modellsystem für die Herstellung hypoallergener Glykoproteine in Pflanzen zu entwickeln. Hierzu diente exemplarisch bislang eine stabile Tabak-Linie (T. Turpen, US-Patent 6,841,659), in der humane Glucocerebrosidase (hCG; EC 3.2.1.45) im Apoplasten akkumuliert. Zusätzliche *GNTI*-RNAi-Drosselung in diesem System führte allerdings dazu, dass hCG nicht mehr nachgewiesen werden konnte (Fig. 8).

In einem anderen Ansatz wurde hGC in einer Karottenzellsuspension produziert (Shaaltiel et al., 2007). Das rekombinante Protein wurde hier mithilfe eines C-terminal angehängten, vakuolären "targeting" Signals (CTTP) zu den Vakuolen transportiert und trägt daher pflanzentypische, immunogene Glykane. Das bei diesem Ansatz benutzte System hat zudem den Nachteil, dass zur Ansteuerung von Vakuolen Extrasequenzen verwendet werden mussten, die für klinische Phase III-Studien nicht erlaubt sind.

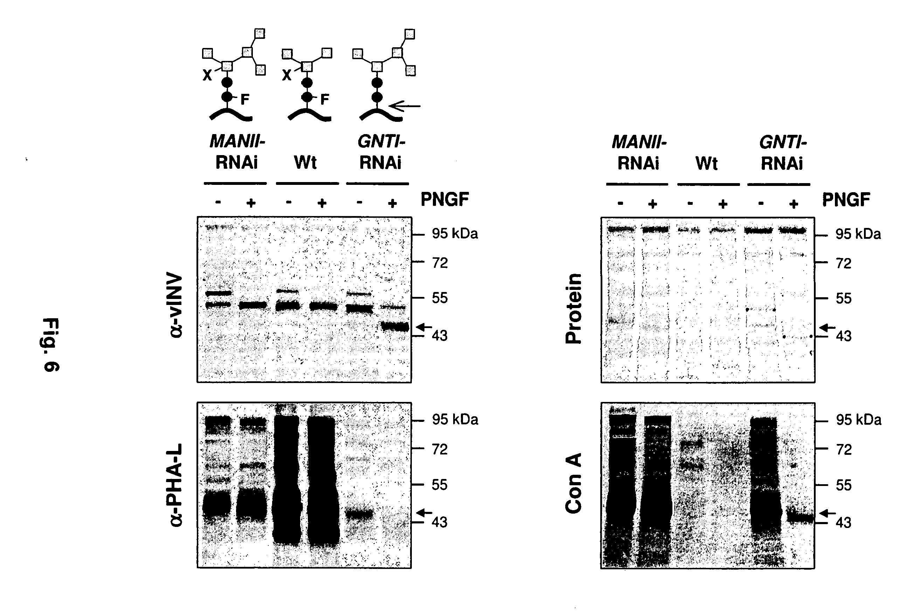

Gezielte Überproduktion von hGC in Tabaksamen resultierte in einer Verminderung der Vitalität des rekombinanten Saatguts. Aus diesem Saatgut erhaltene hGC-Präparationen wiesen zwar reduzierte Gehalte an immunogenen Xylose- und Fucose-Resten auf, dafür wurden aber Galaktose- und Glucose-Modifikationen nachgewiesen. Das beeinträchtigte zwar nicht die Funktionalität der Enzympräparation, minderte jedoch die Aufnahme in Fibroblasten von Gaucher-Patienten (Reggi et al., 2005), wofür Mannose-terminierte Glykane wie bei der vorliegenden Erfindung besser geeignet wären (vgl. anti-PHA-L (Phytohämagglutinin L) zu ConA-Bindung in Fig. 6).

Des Weiteren wurden mittels RNAi-Drosselung in *Nicotiana benthamiana* sowohl "core"-Fucosyltransferasen sowie gleichzeitig Xylosyltransferase dezimiert (Strasser et al., 2008). Ein in diesen Pflanzen erzeugter monoklonaler Antikörper hatte hypoallergene Eigenschaften (Jin et al., 2008). Glykoproteine aus diesen Pflanzen sollten, analog zum Wildtyp (Fig. 6), ebenfalls kaum Mannose-terminierte Glykoproteine aufweisen, was die Wirksamkeit ("clearance") humoral verabreichter hGC in Gaucher-Patienten herabsetzen dürfte.

Für Medizin und Forschung besteht also nach wie vor ein Bedarf für Expressionssysteme bzw. Verfahren, um rekombinante und insbesondere hypoallergene Glykoproteine kostengünstig herstellen zu können. Aufgabe der vorliegenden Erfindung ist daher, ein geeignetes Verfahren bzw. geeignete Pflanzen oder Pflanzenmaterialien zur Erzeugung hypoallergener Glykoproteine, insbesondere für die Therapie lysosomaler Speicherkrankheiten, bereitzustellen.

Diese Aufgabe wird gelöst durch ein Verfahren nach Anspruch 1 sowie mutierte oder transgene Pflanzen bzw. Pflanzenmaterialien nach Anspruch 8 sowie die Verwendung nach Anspruch 11.

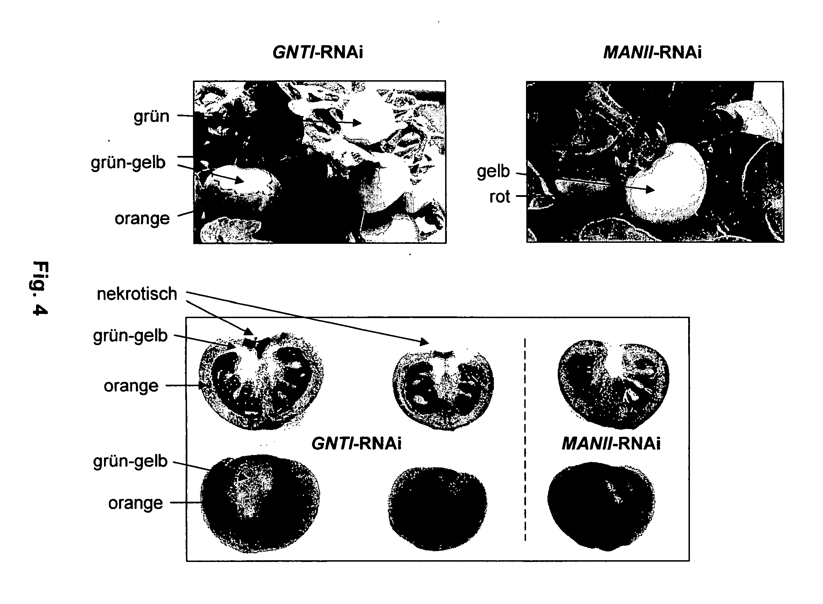

Die gegenwärtigen Erfinder haben überraschend festgestellt, dass Pflanzen, in denen die Aktivität der Golgi α-Mannosidase II (GMII, EC 3.2.1.114) unterdrückt ist, eine signifikante Reduktion der immunologischen Erkennung komplex glykosylierter Proteine in transgenen Pflanzen und damit auch eine generelle Reduktion von CCD-Allergenen ("cross-reactive carbohydrate determinants" der Struktur Man₃XylFucGlcNac₂ oder Man₃XylGlcNac₂) aufweisen, d.h. in hohem Maße hypoallergene Glykoproteine erzeugen. Dies war weitestgehend zwar auch bei parallel untersuchten *GNTI*-RNAi-Linien der Fall, aber im Gegensatz zu den *GNTI*-RNAi-Linien führten *MANII*-RNAi-Linien zu keinerlei Vitalitätseinbußen bei den Pflanzen, in diesem Fall bei Tomaten (Fig. 4).

Dieses Ergebnis war völlig unerwartet, da bisher beschriebene "knock out"-Mutanten von Golgi α-Mannosidase II in *Arabidopsis thaliana N*-Glykane produzierten, die auf der Basis von massenspektrometrischen Glykan-Analysen eine normale Immunogenität erwarten lassen, da sie sowohl "core" Fucose- wie auch Xylose-Reste tragen (Strasser et al., 2006). Der bisherige Stand der Technik ließ es also als höchst unwahrscheinlich erscheinen, dass Pflanzen mit fehlender oder verringerter Aktivität von Golgi α-Mannosidase II geeignet sein könnten, hypoallergene Glykoproteine zu erzeugen.

Die vorliegende Erfindung betrifft somit ein Verfahren zur Erzeugung von hypoallergenen Glykoproteinen, welches das Züchten einer mutierten oder transgenen Pflanze, von Teilen dieser Pflanzen oder daraus hergestellter Pflanzenzellen und das Isolieren eines gewünschten hypoallergenen Glykoproteins aus dem gezüchteten Material umfasst, welches dadurch gekennzeichnet ist, dass die Aktivität des Enzyms Golgi α-Mannosidase II in der mutierten oder transgenen Pflanze, Teilen dieser Pflanzen oder daraus hergestellten Pflanzenzellen beseitigt oder verringert ist.

Teile von solchen Pflanzen können z.B. auch Samen und Vermehrungsmaterial umfassen. Zur Vereinfachung werden Teile von solchen Pflanzen sowie Pflanzenzellen in dieser Anmeldung zusammengefasst auch als Pflanzenmaterial oder Pflanzenmaterialien bezeichnet.

Golgi α-Mannosidase II (GMII, EC 3.2.1.114) ist ein Enzym, das im Golgi-Apparat vielzelliger Eukaryoten lokalisiert ist und die Hydrolyse von verzweigten Mannose-Resten auf dem α1,6-Arm (d.h. von Mannosen in sowohl α1,3- wie α1,6-Verknüpfung) katalysieren kann. Bei Ausschaltung der Golgi α-Mannosidase II bleiben die Mannose-terminierten Glykane auf dem α1,6-Arm erhalten und werden nicht abgespalten, was für die vorliegende Erfindung von Bedeutung ist.

Als Methoden zur Beseitigung oder Verringerung der Aktivität von Golgi α-Mannosidase II kommen effektive "gene silencing"-Ansätze, beispielsweise Cosuppression, Antisense- oder RNAi-Drosselung (Waterhouse et al. 1999), in Betracht. Es können aber ebenfalls sogenannte "knock out"-Mutanten, bei denen das/die Gen(e), welche(s) für Golgi α-Mannosidase II codiert/codieren, gezielt eliminiert oder funktionsunfähig gemacht wurde(n), oder in anderer Weise veränderte Pflanzen, die keine intakte Golgi α-Mannosidase II besitzen, eingesetzt werden.

Die in dem erfindungsgemäßen Verfahren eingesetzten Pflanzen bzw. Pflanzenteile oder Pflanzenzellen können dabei vorher mit dem Gen, welches das gewünschte Glykoprotein codiert, transformiert werden. Verfahren, um solche Gene in Pflanzen einzuführen, sind dem Fachmann bekannt und gehören zum allgemeinen Stand der Technik (z.B. entweder durch T-DNA-Transfer mittels Agrobakterien oder durch direkten Gentransfer in Protoplasten mittels Elektroporation bzw. PEG, sowie auch durch neuere biolistische Methoden nach Beschuss von Pflanzenzellen in ganzen Geweben mit DNA-umhüllten Metallkügelchen ("gene gun", z.B. der Fa. BIORAD)).

Das erzeugte hypoallergene Glykoprotein ist vorzugsweise ein therapeutisches Protein wie z.B. Glucocerebrosidase (Glucosylceramidase; D-Glucosyl-*N*-Acylsphingosin-Glucohydrolase, EC 3.2.1.45), insbesondere humane Glucocerebrosidase (hCG), ein Glykoprotein zur Behandlung der Gaucher-Krankheit, dessen Aufnahme in Leberzellen der Patienten auf terminalen Mannose-Resten beruht (Barton et al., 1990). Ebenso kann das hypoallergene Glykoprotein ein anderes sekretiertes Glykoprotein-Therapeutikum wie z.B. ein Antikörper, ein Interleukin, ein Interferon, eine Lipase etc., insbesondere ein Therapeutikum für eine lysosomale Speichererkrankung sein. Denkbar wäre auch eine Produktion von sekretierten Versionen membranverankerter Enzyme, beispielsweise von α-Mannosidasen (GMII selbst bzw. lysosomaler α-Mannosidase) zur Behandlung von Krankheiten mit HEMPAS-Syndrom (Fukuda, 1990) oder von GnTII zur Behandlung von CDGSII ("carbohydrate deficient glycoprotein syndrome type II"; Tan et al., 1996).

Besonders bevorzugt werden solche Pflanzen oder Pflanzenmaterialien eingesetzt, bei welchen das heterologe Glykoprotein im Apoplasten/der Zellwand oder in Vakuolen akkumuliert. Dadurch wird die Reinigung aus Blättern vereinfacht, da so angereicherte Glykoprotein-Präparationen weniger Verunreinigungen enthalten (siehe z.B. US-Patent Nr. 6,841,659 B2 von Thomas H. Turpen et al.).

Als Pflanzen in dem erfindungsgemäßen Verfahren eignen sich insbesondere die genetische Modellplfanze *Arabidopsis thaliana* oder Solanaceen wie z.B. Tomatenpflanzen (*Lycopersicon spec*.), Tabakpflanzen (*Nicotiana spec*.) oder Kartoffelpflanzen (*Solanum spec*.). Weiterhin sind auch andere agronomisch wichtige Pflanzen wie z.B. Reis und Mais geeignet. Daneben bieten sich als Expressionssysteme die Wasserlinse *Lemna spec.* (Cox et al. 2006) und auch niedere Pflanzen an. Für das Moos *Physcomitrella patens* konnte gezeigt werden, dass "targeted knock-out"-Strategien aufgrund von homologer Rekombination möglich sind (Übersicht in: Decker & Reski, 2007).

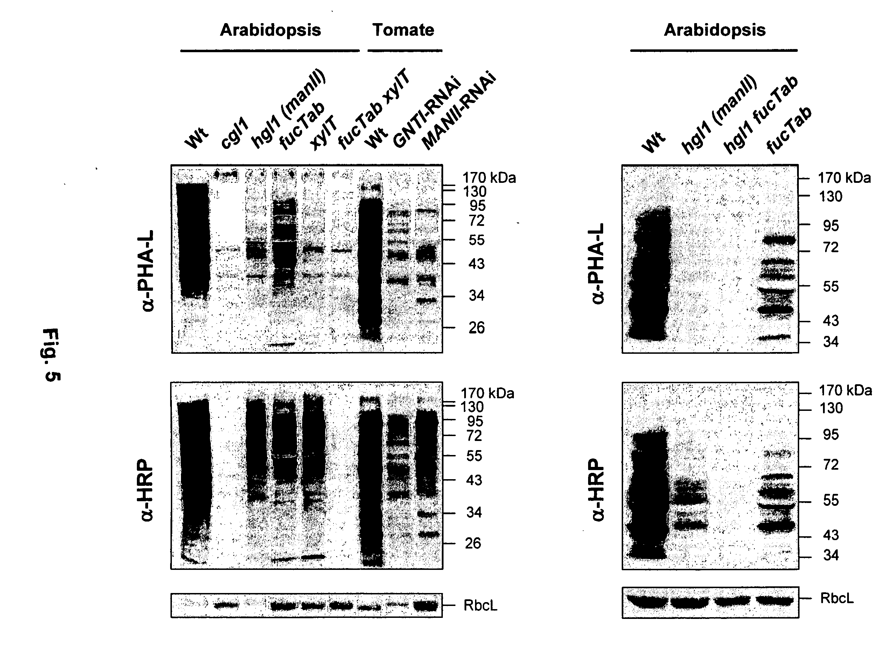

Bei einer besonders bevorzugten Ausführungsform der vorliegenden Erfindung wird zusätzlich die Aktivität von "core"-Fucosyltransferasen beseitigt oder verringert. Enzyme, die den Transfer von "core" α1,3-Fucose-Resten auf Glykoprotein-Glykane katalysieren, d.h. "core" α1,3-Fucosyltransferasen, sind ebenfalls im Golgi-Apparat lokalisiert (Sturm et al., 1987) und benötigen mindestens ein terminales GlcNac, d.h. können frühestens im Anschluss an GnTI arbeiten (Johnson & Chrispeels, 1987). Nachdem durch Kreuzung in Arabidopsis zusätzlich zur Aktivität der GMII (Strasser et al., 2006) auch die Aktivität von zwei "core"-Fucosyltransferase-Isoformen (FucTa und FucTb; Strasser et al., 2004) ausgeschaltet wurde, konnten bei der vorliegenden Erfindung hypoallergene Glykoproteine analog zu *cgl1* (mit GnTI-Defekt) erzielt werden (Fig. 5). Dies sollte ebenfalls einen günstigen Effekt auf die Wirkung von in Pflanzen rekombinant hergestellten Antikörpern haben (ADCC, vgl. Cox et al., 2006; Decker & Reski, 2007; Strasser et al., 2008).

Weiterhin betrifft die vorliegende Erfindung mutierte oder transgene Pflanzen, Teile dieser Pflanzen oder daraus hergestellte Pflanzenzellen, die dadurch gekennzeichnet sind, dass die Aktivität des Enzyms Golgi α-Mannosidase II in der mutierten oder transgenen Pflanze, den Teilen dieser Pflanzen oder daraus hergestellten Pflanzenzellen beseitigt oder verringert ist und diese ein hypoallergenes Glykoprotein produzieren.

Bei besonders bevorzugten transgenen Pflanzen oder Pflanzenmaterialien ist ebenfalls die Aktivität des Enzyms "core"-Fucosyltransferase beseitigt oder verringert.

Die transgenen Pflanzen bzw. Pflanzenmaterialien, die produzierten Glykoproteine, die Methoden zur Beseitigung oder Verringerung der Aktivität von α-Mannosidase II oder "core"-Fucosyltransferase sind vorzugsweise so wie für das erfindungsgemäße Verfahren beschrieben wurde.

Die gegenwärtigen Erfinder haben darüber hinaus bei Versuchen mit Tomaten festgestellt, dass Pflanzen mit verringerter Golgi α-Mannosidase II-Aktivität keine Beeinträchtigungen im Aussehen zeigen und voll ausgereifte rote Früchte ohne Auffälligkeiten bilden. Dagegen wurden bei entsprechenden Pflanzen, in denen anstelle der Aktivität von α-Mannosidase II die Aktivität von GntI verringert wurde, während der Fruchtreife deutliche Phänotypen festgestellt. Besonders auffällig waren hierbei Reife-inhibierte Flecken und nekrotische Stielansätze (vergl. Fig. 4), was vorzeitigen Fruchtfall auslöste. Zusammen mit einer erhöhten Pathogenanfälligkeit der *GNTI-*RNAi-Pflanzen sollten diese Faktoren eine Reinigung und Isolation von heterologen Glykoproteinen erschweren.

Die erfindungsgemäßen transgenen Pflanzen bzw. Pflanzenmaterialien eignen sich daher insbesondere sowohl zur Erzeugung hypoallergener heterologer Glykoproteine als auch als hypoallergene Pflanzen, die als Nahrungsmittel für Allergiker geeignet sind, für die CCD-Epitope ("cross-reactive carbohydrate determinants") ein Problem darstellen. Die Erfindung betrifft damit auch hypoallergene Pflanzen bzw. Pflanzenmaterialien als solche.

Die Eignung kann noch dadurch erhöht werden, dass neben der Aktivität von Golgi α-Mannosidase II zusätzlich die Aktivität von "core"-Fucosyltransferase beseitigt, gehemmt oder verringert wird.

Schließlich sind auch die Glykoproteine, die mittels der erfindungsgemäßen Verfahren oder aus den erfindungsgemäßen Pflanzen bzw. Pflanzenmaterialien erhältlich sind, Teil der vorliegenden Erfindung.

Die Erfindung wird nun anhand von Ausführungsbeispielen mit Bezug auf die beigefügten Figuren näher erläutert. Die Ausführungsbeispiele sollen den Umfang der Erfindung aber nicht beschränken.

### Die Figuren zeigen:

Fig. 1: Reihenfolge Pflanzen-typischer *N*-Glykan-Modifikationen, denen sekretorische Glykoproteine bei Passage durch das ER und den Golgi-Apparat unterliegen. Glykane, die bei Defekten in α-Mannosidase II akkumulieren, sind als Weg mit gestrichelten Pfeilen dargestellt.

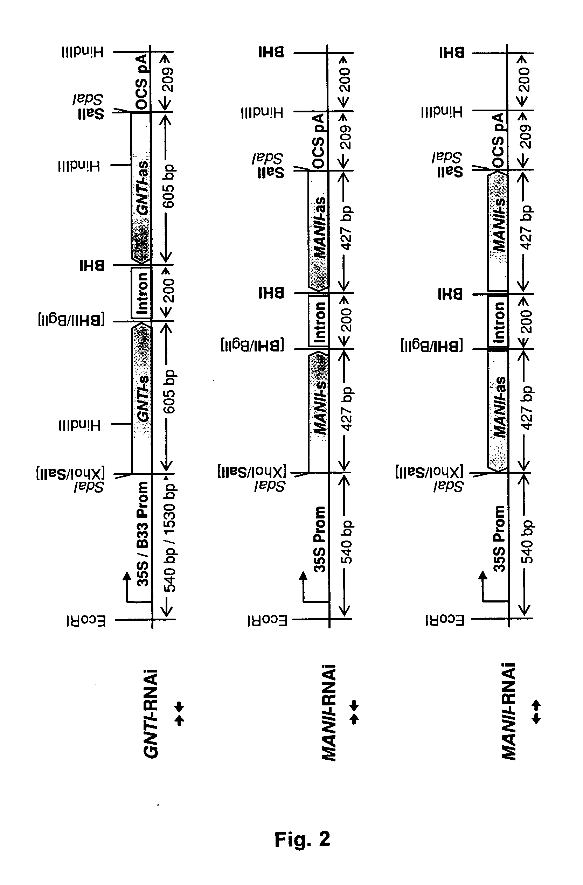

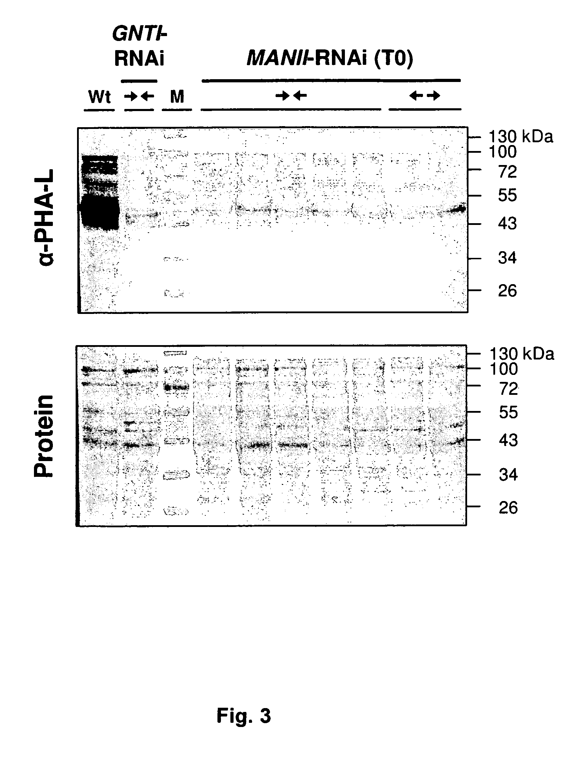

Fig. 2: Schema der verwendeten RNAi-Konstrukte. Für den *MANII*-RNAi-Ansatz wurden unterschiedliche Orientierungen der cDNA-Fragmente in sense (s) und antisense (as) relativ zum zentralen Intron getestet (Ergebnis, vgl. Fig. 3), dabei erfolgte die Klonierung des dsRNAi-Konstrukts analog zu Chen et al. (2003), mit anschließender Insertion in die Pflanzen-Expressionskassette wie bei von Schaewen et al. (1990). Abk.: 35S, Promotor aus BlumenkohlMosaikvirus (konstitutiv); B33, Promotor für *Solanaceen* (Knolle/Frucht-spezifisch); OCS pA, Polyadenylierungssignal der Octopin-Synthase (aus Agrobakterien T-DNA).

Fig. 3: Immunoblot-Detektion des CCD-Musters (mit PHA-L-Antiserum) in Tomatenfrucht-Extrakten der *MANII*-RNAi Primär-Transformanten (T0) mit Wildtyp (Wt) und *GNTI*-RNAi-Linien zum Vergleich (oben). Die Ponceau S reversibel-gefärbte Membran (unten) zeigt, dass vergleichbare Proteinmengen transferiert wurden. M, Markerproteine (Pre-stained Protein Ladder, Fermentas). Dies zeigt für die vorliegende Erfindung, dass die CCD-Drosselung in *MANII*-RNAi-Linien mit der in *GNTI*-RNAi-Linien vergleichbar ist (bei unterschiedlichem Laufverhalten der verbleibenden, CCD-reaktiven Glykoproteine).

Fig. 4: Vergleich von hypoallergenen Tomatenfrüchten. Während *GNTI*-RNAi-Linien einen Reifephänotyp zeigen (fleckige, unreife Stellen und nekrotische Stielansätze), bilden *MANII-*RNAi-Linien vollreife Früchte aus. Dies zeigt für die vorliegende Erfindung, dass die CCD-Drosselung in *MANII*-RNAi-Linien die Fruchtentwicklung phänotypisch nicht beeinträchtigt.

Fig. 5: CCD-Muster in Blatt-Extrakten von ausgewählten Arabidopsis *N*-Glykosylierungsmutanten und Tomaten RNAi-Linien bei Immunodetektion mit PHA-L-Antiserum, welches überwiegend Xylose-Reste erkennt (Laurière et al., 1989) und - nach Strippen des Blots - mit HRP-Antiserum (stärkere "core"-Fucose-Erkennung). Der Vergleich der beiden linken Blots zeigt für die Arabidopsis-Mutanten, dass *hgl1* (*manII*) mit *xylT* vergleichbar ist und deutet an, dass Restreaktivität der Tomaten *MANII*-RNAi-Extrakte auf "core"-Fucosen beruhen muss. Rechts, nach Kreuzung von *hgl1* (*manII*) und *fucTa fucTb* ergibt sich eine weitere Reduktion der CCD-Erkennung in Arabidopsis *manII fucTab*-Tripelmutanten (ohne "core"-Fucose-Reste). Wie die Blot-Entwicklung mit HRP-Antiserum (unten) zeigt, impliziert dies für die vorliegende Erfindung, dass zusätzliche Drosselung von "core"-Fucosyltransferase-Aktivität die CCD-Reaktivität der *MANII*-RNAi-Linien noch weiter reduzieren sollte.

Fig. 6: Tomatenfrucht-Extrakte von Wildtyp und RNAi-Linien ohne/mit PNGase F-Behandlung. Die Glykoproteine der *MANII*-RNAi-Linien (Beispiel: vakuoläre Invertase, anti-vINV) tragen "core"-Fucose-Reste (PNGase F kann nicht spalten) und ungetrimmte Mannose-Reste auf dem α1,6-Mannose-Arm, was in stärkerer ConA-Bindung im Vergleich zum Wildtyp resultiert. Dies zeigt für die vorliegende Erfindung, dass Glykoproteine aus *hgl1* (*manII*)-Mutanten oder *MANII-*RNAi-Linien für eine bevorzugte Aufnahme in Zellen von Patienten mit lysosomalen Speicherkrankheiten (wie Gaucher, etc.) geeignet sind.

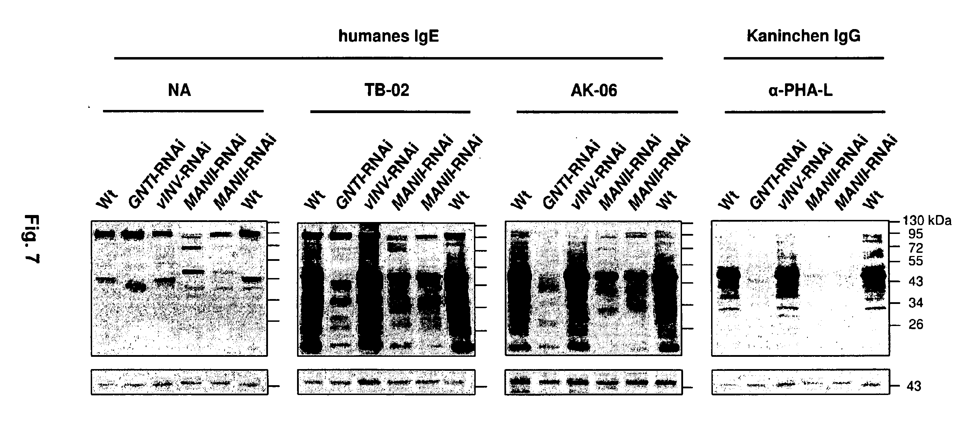

Fig. 7: Immunoblot-Analyse von Tomatenfrucht-Extrakten mit Patientenseren. Der chemilumineszente Nachweis Allergie-Typ I-relevanter Immunglobuline (IgE) zeigt reduzierte Reaktivität der beiden CCD-Allergiker mit Proteinen aus *MANII*-RNAi-Linien, die vergleichbar mit *GNTI*-RNAi-Linien sind. Die colorimetrische Überentwicklung des Nicht-Allergiker (NA)-Blots mit PHA-L-Antiserum (Kaninchen IgG) ergibt ein vergleichbares Muster (Kontrolle). Darunter, zum Vergleich der Proteinmengen, 43 kDa-Region der Ponceau S-gefärbten Blots. Abk.: NA, Nicht-Allergiker; TB-02 und AK-06, CCD-Allergiker. Dies zeigt für die vorliegende Erfindung, dass CCD-Drosselung via *MANII*-RNAi für CCD-Allergiker immunologisch relevant ist.

Fig. 8: Nachweisbarkeit des hGC-Signals in Abhängigkeit von der *N*-Glykan-Dekoration in Apoplasten-Eluaten aus Blättern von Tabak RNAi-Linien. Zwei Doppelpunkte stehen für stabile Integration der jeweiligen Konstrukte in das Pflanzengenom. X, Nachkommen, die aus Kreuzungen der entsprechenden Linien resultieren. MD und Xan (Xanthi) bezeichnen Tabak-Sorten (*Nicotiana tabacum*). His-hGC, rekombinante humane Glucocerebrosidase mit N-terminalem His-"tag" ohne *N*-Glykane (nach Überexpression in *E. coli* und anschließender Affinitätsreinigung). Die Pfeile markieren die Position der in Pflanzen produzierten hGC-Glykoproteine auf dem Blot, die nur in der hypoallergenen *MANII*-RNAi-Linie Nr. 1 deutlich nachweisbar sind. Für die vorliegende Erfindung zeigt die fehlende Bindung von PHA-L-Antiserum, dass hGC in *MANII*-RNAi-Linien hypoallergene N-Glykane trägt.

### Beispiele

### Beispiel 1. Erzeugung von Tomatenfrüchten mit verringerter Aktivität von Golgi α-Mannosidase II (MANII-RNAi-Linien)

Herstellung von RNAi-Linien: Die *MANII*-dsRNAi-Konstrukte wurden auf Grundlage eines Tomaten-EST-Klons (TA33056_4081 aus der Datenbank TIGR) speziell für Tomate konzipiert. Ein ca. 400 bp-Fragment der Golgi α-Mannosidase II wurde mittels RT-PCR aus Blatt-RNA von *Lycopersicon esculentum* der Sorte Moneymaker "Microtom" gewonnen. Dieses Fragment wurde in den Vektor pUC-RNAi (Chen et al., 2003) zweimal flankierend zum ersten Intron der Kartoffel GA20-Oxidase über SaII/BamHI, bzw. XhoI/BglII *via* kompatible Überhänge inseriert. So wurden zwei verschiedene *MANII*-dsRNAi-Konstrukte hergestellt. Ein "sense"-Intron-"antisense"-Konstrukt mit den Primern:
"Sense" 5'-CACCGTCGACAGTCCAAGCACATCCTAGATA-3' (SalI unterstrichen) (SEQ ID NO:1)
"Antisense" 5'-NNNGGATCCAAATTCTGGTTTAAAGCCA-3' (BamHI unterstrichen) (SEQ ID NO:2)

Sowie ein "antisense"-Intron-"sense"-Konstrukt mit den Primern:
"Sense" 5'-CACCGGATCCAAGCACATCCTAGATATGTTG-3' (BamHI unterstrichen) (SEQ ID NO:3)
"Antisense" 5'-NNNGTCGACCAAATTCTGGTTTAAAGCCA-3' (SalI unterstrichen) (SEQ ID NO:4)

Anschließend wurden die dsRNAi-Bereiche mit PstI ausgeschnitten und in eine Expressionskassette (zwischen den konstitutiven CaMV 35S-Promotor und das OCS-Polyadenylierungssignal) des mit SdaI geöffneten binären Vektors pBinAR(HygR; Becker, 1990) inseriert.

Mit den binären Plasmid-Konstrukten wurden kompetente GV2260 Agrobakterienzellen (Stamm C58C1 mit Virulenzplasmid pGV2260, Deblaere et al., 1985) direkt transformiert (Höfgen und Willmitzer, 1988) und zur Cokultivierung von Tomaten-Kotyledonen auf einem "feeder layer" aus Tabak BY2-Zellen verwendet, wie bei Ling et al. (1998) beschrieben. Die Regeneration von *MANII*-RNAi-Transformanten erfolgte unter Selektionsdruck (10 mg/l Hygromycin B).

Die erhaltenen Tomatenfrüchte zeigten keinerlei Flecken oder Vitalitätseinbußen und erwiesen sich bei *in vitro*-Analysen für einige Allergiepatienten (CCD-Allergiker, siehe Fig. 7) als weitgehend hypoallergen.

### Beispiel 2. Isolation von (Glyko-) Proteinen aus Tomatenfrüchten

Protein-Extraktion: Zunächst wurden die Samen aus frischen Tomatenfrüchten entfernt und die verbleibende Frucht entweder komplett oder in Fruchtfleisch und Schale getrennt in flüssigem Stickstoff zu einem feinen Pulver gemörsert und bis zur weiteren Verwendung aliquotiert bei -80°C gelagert. Zur Extraktion wurde das gemörserte Material mit eiskaltem Puffer (entweder 100 mM HEPES pH 7,5 oder 50 mM HEPES pH 7,5 mit 250 mM NaCl) und weiteren Zusätzen (2 mM Na₂S₂O₅, 1 mM Pefabloc SC, SERVA, Proteinase-Inhibitor-Cocktail, SIGMA, 1:5000) extrahiert und anschließend 10 min bei 4°C zentrifugiert. Die Überstände wurden nach Bestimmung des Proteingehalts (Bradford, 1976) für Immunoblots verwendet.

### Beispiel 3: Untersuchung des isolierten Glykoproteins mit verschiedenen Antiseren - Immunoblot-Analysen

Protein-Extrakte wurden unter reduzierenden Bedingungen in 11-15%igen Polyacrylamid-Gelen mittels SDS-PAGE aufgetrennt (Laemmli, 1970) und auf Nitrozellulosemembranen (0,45 µm PROTRAN, Schleicher&Schüll) in einer Nass-Zelle (Mini-Protean 3-System, Bio-Rad) für 2 Stunden bei 350 mA transferiert. Auf der Membran wurden die Proteine mit Ponceau S (0,3% in 3% TCA, SERVA) gefärbt und fixiert, und nach der Dokumentation (Flachbett-Scanner) mit TBST (20 mM Tris, 150 mM NaCl, 0,1% (v/v) Tween 20, pH 7,4) wieder entfärbt. Die Blot-Membranen wurden anschließend mit 2% Milchpulver in TBST entweder über Nacht bei 4°C oder mindestens für 1 Stunde bei RT blockiert.

Für chemilumineszente Entwicklungen wurden die Blots in verdünnten Antiseren (1:5.000 in TBST, 2% Milchpulver) unter Schütteln für 2 Stunden bei RT inkubiert. Anschließend erfolgte 3maliges Waschen mit TBST und eine weitere Inkubation mit HRP-konjugiertem "goat-anti-rabbit" IgG (Bio-Rad, 1:10.000 in TBST, 2% Milchpulver) für 1 Stunde, und abschließendem 3maligen Waschen. Entwicklung und anschließendes Strippen der Blot-Membranen erfolgte nach Herstellerangaben für das ECL Advance Western Blotting Detection Kit (GE Healthcare).

Zur Markierung von pflanzlichen Protein-Glykanen (CCD) wurde routinemäßig ein Kaninchen-Antiserum verwendet, das gegen PHA-L hergestellt wurde (Laurière et al., 1989) und neben "core"-Fucose-Resten überwiegend Xylose-Reste erkennt (1:10.000 in TBST, 2% Milchpulver für 2 Stunden). Alternativ wurde ein käufliches Kaninchen-Antiserum gegen HRP (Sigma) verwendet (1:20.000 in 40 mM Tris pH 7,4, 300 mM NaCl, 0,1% (v/v) Tween 20, 2% Milchpulver für 2 Stunden), bei dem die "core"-Fucose-Erkennung bedingt durch Besonderheiten des HRP-Glykoproteins (Wuhrer et al., 2004; 2005) in Pflanzenextrakten erhöht ist.

Zur Markierung von vakuolärer Invertase (vINV) und humaner Glucocerebrosidase (hGC) wurden Antiseren verwendet, die nach Immunisierung von Kaninchen mit N-terminal verkürzten Versionen und His-"tag" erhalten wurden (zunächst Klonieren entsprechender cDNA-Fragmente in pET16b, gefolgt von IPTG-induzierter Überexpression in *E. coli* BL21-Zellen (Novagen) und anschließender Affinitätsreinigung an Ni-NTA (Qiagen)). Alle weiteren Schritte wurden wie oben beschrieben durchgeführt.

Für colorimetrische Entwicklungen wurden die Antiseren 10fach konzentrierter eingesetzt, mit HRP-konjugiertem "goat-anti-rabbit" IgG (Bio-Rad, 1:3.000 in TBST, 2% Milchpulver) 1 Stunde bei RT inkubiert und wie bei von Schaewen et al. (1993) beschrieben detektiert.

IgE-Immunoblots: Für den Immunoblot-Nachweis von IgE-Antikörpern aus humanen Seren wurden die blockierten Blot-Membranen mit verdünnten Patienten-Seren (1:10 in TBST, 2% Milchpulver) unter Schütteln für 3 Stunden bei RT inkubiert, 3mal mit TBST gewaschen und anschließend mit "affinity-purified antibody peroxidase-labeled goat-anti-human" IgE(ε) (Kirkegaard & Perry Laboratories, MD, USA) (1:10.000 in TBST) für 1 Stunde inkubiert und wie oben gewaschen. Die anschließende chemilumineszente Entwicklung erfolgte ebenfalls mit dem ECL Advance Western Blotting Detection Kit (GE Healthcare) nach Herstellerangaben.

Die PNGase F-Behandlung der Tomatenfrucht-Extrakte folgte den Herstellerangaben (Roche). Die ConA-Affinoblot-Entwicklung wurde analog zu Faye & Chrispeels (1985) mit 10fach geringeren Konzentrationen an Concanavalin A (ConA, Sigma) und HRP (Fluka) für die ECL-Entwicklung durchgeführt.

### Zitierte Literatur

Barton NW, Furbish FS, Murray GJ, Garfield M, Brady RO (1990). Therapeutic response to intravenous infusions of glucocerebrosidase in a patient with Gaucher disease. Proc. Natl. Acad. Sci. USA. 87: 1913-1916.
Becker D (1990). Binary vectors, which allow the exchange of plant selectable markers and reporter genes. Nucl. Acids Res. 18: 203.
Bradford MM (1976). A rapid and sensitive method for the quantitation of microgram quantities of protein utilizing the principle of protein-dye binding. Anal. Biochem. 72: 248-254.
Chen S, Hofius D, Sonnewald U, Börnke F (2003). Temporal and spatial control of gene silencing in transgenic plants by inducible expression of double-stranded RNA. Plant J. 36: 731-740.
Chui D, Oh-Eda M, Liao YF, Panneerselvam K, Lal A, Marek KW, Freeze HH, Moremen KW, Fukuda MN, Marth JD (1997). Alpha-mannosidase-II deficiency results in dyserythropoiesis and unveils an alternate pathway in oligosaccharide biosynthesis. Cell 90: 157-167.
Cox KM, Sterling JD, Regan JT, Gasdaska JR, Frantz KK, Peele CG, Black A, Passmore D, Moldovan-Loomis C, Srinivasan M, Cuison S, Cardarelli PM, Dickey LF (2006). Glycan optimization of a human monoclonal antibody in the aquatic plant Lemna minor. Nat. Biotechnol. 24: 1591-1597.
Deblaere R, Bytebier B, De Greve H, Debroeck F, Schell J, van Montagu M, Leemans J (1985). Efficient octopine Ti plasmid-derived vectors of Agrobacterium-mediated gene transfer to plants. Nucl. Acids Res. 13: 4777-4788.
Decker EL, Reski R (2007). Moss bioreactors producing improved biopharmaceuticals. Curr. Opin. Biotechnol. 18: 393-398. Review.
Faye L, Chrispeels MJ (1985). Characterization of N-linked oligosaccharides by affinobloting with concanavalin A-peroxidase and treatment of the blots with glycosidases. Anal. Biochem. 149: 218-224.
Faye L, Chrispeels MJ (1988). Common antigenic determinants in the glycoproteins of plants, molluscs, and insects. Glycoconj. J. 5: 245-256.
Fukuda MN (1990). HEMPAS disease: genetic defect of glycosylation. Glycobiology 1: 9-15. Review.
Höfgen R, Willmitzer L (1988). Storage of competent cells for Agrobacterium transformation. Nucl. Acids Res. 16: 9877.
Ioffe E, Stanley P (1994). Mice lacking N-acetylglucosaminyltransferase I activity die at midgestation, revealing an essential role for complex or hybrid N-linked carbohydrates. Proc. Natl. Acad. Sci. USA 91: 728-732.
Jin C, Altmann F, Strasser R, Mach L, Schähs M, Kunert R, Rademacher T, Glössl J, Steinkellner H (2008). A plant-derived human monoclonal antibody induces an anticarbohydrate immune response in rabbits. Glycobiology 18: 235-241.
Johnson KD, Chrispeels MJ (1987). Substrate specificities of N-acetylglucosaminyl-, fucosyl-, and xylosyltransferases that modify glycoproteins in the Golgi apparatus of bean cotyledons. Plant Physiol. 84: 1301-1308.
Laemmli UK (1970). Cleavage of structural proteins during the assembly of the head of bacteriophage T4. Nature 227: 680-685.
Lauriere M, Lauriere C, Chrispeels MJ, Johnson KD, Sturm A (1989). Characterization of a xylose-specific antiserum that reacts with the complex asparagine-linked glycans of extracellular and vacuolar glycoproteins. Plant Physiol. 90: 1182-1188.
Li D, Li Y, Wu X, Li Q, Yu J, Gen J, Zhang XL (2008). Knockdown of Mgat5 inhibits breast cancer cell growth with activation of CD4+ T cells and macrophages. J. Immunol. 180: 3158-3165.
Ling H-Q, Kriseleit D, Ganal MW (1998). Effect of ticarcillin/potassium clavulanate on callus growth and shoot regeneration in Agrobacterium-mediated transformation of tomato (Lycopersicon esculentum Mill.). Plant Cell Rep. 17: 843-847.
Metzler M, Gertz A, Sarkar M, Schachter H, Schrader JW, Marth JD (1994). Complex asparagine-linked oligosaccharides are required for morphogenic events during postimplantation development. EMBO J. 13: 2056-2065.
Moremen KW (2002). Golgi alpha-mannosidase II deficiency in vertebrate systems: implications for asparagine-linked oligosaccharide processing in mammals. Biochim. Biophys. Acta 1573: 225-235.
Reggi S, Marchetti S, Patti T, De Amicis F, Cariati R, Bembi B, Fogher C (2005). Recombinant human acid beta-glucosidase stored in tobacco seed is stable, active and taken up by human fibroblasts. Plant Mol. Biol. 57: 101-113.
Shaaltiel Y, Bartfeld D, Hashmueli S, Baum G, Brill-Almon E, Galili G, Dym O, Boldin-Adamsky SA, Silman I, Sussman JL, Futerman AH, Aviezer D (2007). Production of glucocerebrosidase with terminal mannose glycans for enzyme replacement therapy of Gaucher's disease using a plant cell system. Plant Biotechnol. J. 5: 579-590.
Strasser R, Altmann F, Mach L, Glössl J, Steinkellner H (2004). Generation of Arabidopsis thaliana plants with complex N-glycans lacking betal,2-linked xylose and core alphal,3-linked fucose. FEBS Lett. 561: 132-136.
Strasser R, Schoberer J, Jin C, Glössl J, Mach L, Steinkellner H (2006). Molecular cloning and characterization of Arabidopsis thaliana Golgi alpha-mannosidase II, a key enzyme in the formation of complex N-glycans in plants. Plant J. 45: 789-803.
Strasser R, Stadlmann J, Schähs M, Stiegler G, Quendler H, Mach L, Glössl J, Weterings K, Pabst M, Steinkellner H (2008). Generation of glyco-engineered Nicotiana benthamiana for the production of monoclonal antibodies with a homogeneous human-like N-glycan structure. Plant Biotechnol. J. 6: 392-402.
Sturm A, Johnson KD, Szumilo T, Elbein AD, Chrispeels MJ (1987). Subcellular localization of glycosidases and glycosyltransferases involved in the processing of N-linked oligosaccharides. Plant Physiol. 85: 741-745.
Tan J, Dunn J, Jaeken J, Schachter H (1996). Mutations in the MGAT2 gene controlling complex N-glycan synthesis cause carbohydrate-deficient glycoprotein syndrome type II, an autosomal recessive disease with defective brain development. Am. J. Hum. Genet. 59: 810-807.
von Schaewen A, Stitt M, Schmidt R, Sonnewald U, Willmitzer L (1990). Expression of a yeast-derived invertase in the cell wall of tobacco and Arabidopsis plants leads to accumulation of carbohydrate and inhibition of photosynthesis and strongly influences growth and phenotype of transgenic tobacco plants. EMBO J. 9: 3033-3044.
von Schaewen A, Sturm A, O'Neill J, Chrispeels MJ (1993). Isolation of a mutant Arabidopsis plant that lacks N-acetyl glucosaminyl transferase I and is unable to synthesize Golgi-modified complex N-linked glycans. Plant Physiol. 102: 1109-1118.
Waterhouse PM, Smith NA, Wang MB (1999). Virus resistance and gene silencing: killing the messenger. Trends Plant Sci. 4: 452-457.
Wenderoth I, von Schaewen A (2000). Isolation and characterization of plant N-acetyl glucosaminyltransferase I (GntI) cDNA sequences. Functional analyses in the Arabidopsis cgl mutant and in antisense plants. Plant Physiol. 123: 1097-1108.
Wuhrer M, Hokke CH, Deelder AM (2004). Glycopeptide analysis by matrix-assisted laser desorption/ionization tandem time-of-flight mass spectrometry reveals novel features of horseradish peroxidase glycosylation. Rapid Commun. Mass Spectrom. 18: 1741-1748.
Wuhrer M, Balog CI, Koeleman CA, Deelder AM, Hokke CH (2005). New features of sitespecific horseradish peroxidase (HRP) glycosylation uncovered by nano-LC-MS with repeated ion-isolation/fragmentation cycles. Biochim. Biophys. Acta 1723: 229-239.

## Patentansprüche

1. Verfahren zur Erzeugung von hypoallergenen Glykoproteinen, umfassend das Züchten einer mutierten oder transgenen Pflanze, von Teilen dieser Pflanzen oder daraus hergestellter Pflanzenzellen und das Isolieren eines gewünschten hypoallergenen Glykoproteins aus dem gezüchteten Material, **dadurch gekennzeichnet, dass** die Aktivität des Enzyms Golgi α-Mannosidase II in der mutierten oder transgenen Pflanze, den Teilen dieser Pflanzen oder daraus hergestellten Pflanzenzellen beseitigt oder verringert ist.

2. Verfahren nach Anspruch 1, **dadurch gekennzeichnet, dass** die Aktivität des Enzyms Golgi α-Mannosidase II durch Mutation oder "gene silencing", wie z.B. Cosuppression, Antisense- oder RNAi-Drosselung, beseitigt oder verringert wurde.

3. Verfahren nach Anspruch 1 oder Anspruch 2, **dadurch gekennzeichnet, dass** die mutierte oder transgene Pflanze, Teile dieser Pflanzen oder daraus hergestellte Pflanzenzellen zusätzlich mit einem Gen, welches das gewünschte hypoallergene Glykoprotein codiert, transformiert wurde/wurden.

4. Verfahren nach einem der Ansprüche 1 bis 3, **dadurch gekennzeichnet, dass** das hypoallergene Glykoprotein Glucocerebrosidase oder ein anderes sekretiertes Glykoprotein-Therapeutikum ist.

5. Verfahren nach einem der Ansprüche 1 bis 4, **dadurch gekennzeichnet, dass** das hypoallergene Glykoprotein im pflanzlichen Apoplasten/der Zellwand oder in Vakuolen akkumuliert und daraus isoliert werden kann.

6. Verfahren nach einem der Ansprüche 1 bis 5, **dadurch gekennzeichnet, dass** die mutierte oder transgene Pflanze, Teile dieser Pflanzen oder daraus hergestellte Pflanzenzellen abgeleitet sind von Solanaceen wie z.B. Tomatenpflanzen, Kartoffelpflanzen oder Tabakpflanzen oder weiteren agronomisch wichtigen Pflanzen wie z.B. Reis oder Mais, oder auch Arabidopsis.

7. Verfahren nach einem der Ansprüche 1 bis 6, **dadurch gekennzeichnet, dass** zusätzlich die Aktivität des Enzyms "core"-Fucosyltransferase in der mutierten oder transgenen Pflanze, den Teilen dieser Pflanzen oder daraus hergestellten Pflanzenzellen beseitigt oder verringert ist.

8. Mutierte oder transgene Pflanze, Teile dieser Pflanzen oder daraus hergestellte Pflanzenzellen, **dadurch gekennzeichnet, dass** die Aktivität des Enzyms Golgi α-Mannosidase II in der mutierten oder transgenen Pflanze, den Teilen dieser Pflanzen oder den daraus hergestellten Pflanzenzellen beseitigt oder verringert ist und diese ein hypoallergenes Glykoprotein produzieren.

9. Mutierte oder transgene Pflanze, Teile dieser Pflanzen oder daraus hergestellte Pflanzenzellen nach Anspruch 8, **dadurch gekennzeichnet, dass** zusätzlich die Aktivität des Enzyms "core"-Fucosyltransferase in der mutierten oder transgenen Pflanze, den Teilen dieser Pflanzen oder daraus hergestellten Pflanzenzellen beseitigt oder verringert ist.

10. Mutierte oder transgene Pflanze, Teile dieser Pflanzen oder daraus hergestellte Pflanzenzellen nach Anspruch 8 oder Anspruch 9, **dadurch gekennzeichnet, dass** diese hypoallergen ist/sind.

11. Verwendung einer mutierten oder transgenen Pflanze, von Teilen dieser Pflanzen oder daraus hergestellten Pflanzenzellen nach einem der Ansprüche 8 bis 10 zur Erzeugung hypoallergener Glykoproteine und/oder hypoallergener Pflanzen.

12. Hypoallergene Glykoproteine, erhältlich durch ein Verfahren nach einem der Ansprüche 1 bis 7, erhältlich aus einer mutierten oder transgenen Pflanze, Teilen dieser Pflanzen oder daraus hergestellten Pflanzenzellen nach einem der Ansprüche 8 bis 10 oder erhältlich durch eine Verwendung nach Anspruch 11.


SEQUENZPROTOKOLL

<110> Westfälische Wilhelms-Universität Münster

<120> Verfahren zur Erzeugung von hypoallergenen Glykoproteinen in
mutierten oder transgenen Pflanzen oder Pflanzenzellen sowie
mutierte oder transgene Pflanzen und Pflanzenzellen zur Erzeugung
hypoallergener Glykoproteine

<130> P1048EP00

<160> 4

<170> PatentIn Version 3.3

<210> 1
<211> 31
<212> DNA
<213> Künstliche Sequenz

<400> 1
caccgtcgac agtccaagca catcctagat a 31

<210> 2
<211> 28
<212> DNA
<213> Künstliche Sequenz

<220>
<221> misc_feature
<222> (1)..(3)
<223> n ist a, c, g oder t

<400> 2
nnnggatcca aattctggtt taaagcca 28

<210> 3
<211> 31
<212> DNA
<213> Künstliche Sequenz

<400> 3
caccggatcc aagcacatcc tagatatgtt g 31

<210> 4
<211> 29
<212> DNA

<213> Künstliche Sequenz

<220>
<221> misc_feature
<222> (1)..(3)
<223> n ist a, c, g oder t

<400> 4
nnngtcgacc aaattctggt ttaaagcca 29